Europäisches Patentamt

European Patent Office

Office européen des brevets

(19)

(11) Veröffentlichungsnummer: **0 190 422**
**B1**

(12) **EUROPÄISCHE PATENTSCHRIFT**

(45) Veröffentlichungstag der Patentschrift:
29.03.89

(51) Int. Cl.⁴: **A 61 F 2/30,** A 61 F 2/34

(21) Anmeldenummer: **85115115.9**

(22) Anmeldetag: **28.11.85**

(54) Knochenimplantat aus Kunststoff.

(30) Priorität: **25.01.85 CH 328/85**

(43) Veröffentlichungstag der Anmeldung:
**13.08.86 Patentblatt 86/33**

(45) Bekanntmachung des Hinweises auf die Patenterteilung:
**29.03.89 Patentblatt 89/13**

(84) Benannte Vertragsstaaten:
**AT DE FR GB IT**

(56) Entgegenhaltungen:
**EP-A-0 016 480**
**EP-A-0 038 902**
**CH-A-611 794**
**DE-A-3 130 732**
**DE-A-3 228 113**
**FR-A-2 548 533**
**US-A-4 479 271**
**US-A-4 492 577**

(73) Patentinhaber: **GEBRÜDER SULZER
AKTIENGESELLSCHAFT, Zürcherstrasse 9, CH-
8401 Winterthur (CH)**

(72) Erfinder: **Frey, Otto, Wallrütistrasse 56, CH- 8400
Winterthur (CH)**
Erfinder: **Semlitsch, Manfred, Dr., Endlikerstrasse
86, CH- 8400 Winterthur (CH)**
Erfinder: **Weber, Heinz, Bulligerstrasse 1, CH- 8400
Winterthur (CH)**

(74) Vertreter: **Dipl.- Ing. H. Marsch Dipl.- Ing. K.
Sparing Dipl.- Phys.Dr. W.H. Röhl
Patentanwälte, Rethelstrasse 123, D-4000
Düsseldorf 1 (DE)**

## Beschreibung

Die Erfindung betrifft ein Knochenimplantat aus Kunststoff, insbesondere eine Hüftgelenkspfanne, dessen den Knochen kontaktierende Oberfläche mindestens teilweise mit einer eine zementfreie Verankerung fördernden porösen Struktur versehen ist.

Für eine zementfreie Verankerung von Endoprothesen-Teilen ist es bekannt (DE-A-3 130 732), die Kontaktfläche zum Knochen von Kunststoffimplantaten mit einer Vielzahl von Makro- und Mikroporen zu versehen; die poröse Schicht kann dabei mit einem Bewehrungsnetz aus einem Metallgitter stabilisiert sein. Weiterhin ist aus dem US-Patent 4 479 271 ein Tibiateil einer Kniegelenkprothese bekannt, bei dem ein Kunststoffkörper und Metallkörper aus, wie Filz, regellos zusammengepressten Metallfasern durch teilweises Einbetten des Filzes in die Kunststoffoberfläche miteinander verbunden sind.

Weder für das Einwachsen von Knochengewebe noch für das Einbetten in den Kunststoff sind bei den Konstruktionen nach den beiden genannten Veröffentlichungen über die ganze Grenzfläche zwischen porösem Körper und Knochengewebe bzw. Kunststoff definierte, gleichmässige und kontrollierbare Poren vorhanden. Weiterhin kann bei ihnen eine aus medizinischen Gründen häufig geforderte vollständige Trennung des Knochengewebes vom Kunststoff nicht gewährleistet werden.

Aufgabe der Erfindung ist es daher, ein Kunststoffimplantat zu schaffen, bei dem die vorstehend geschilderten Nachteile der bekannten Konstruktionen vermieden sind.

Diese Aufgabe wird mit der Erfindung dadurch gelöst, dass die poröse Struktur eine mehrlagige Maschenware aus Metalldraht mit in jeder Lage definierten und regelmässigen Maschenweiten ist, wobei mindestens eine Lage in die Kontaktfläche des Kunststoffimplantates mindestens teilweise eingebettet ist und auf der mindestens teilweise eingebetteten Lage mehrere Lagen der gleichen Maschenware angeordnet sind, deren Maschenweiten von Lage zu Lage nach aussen zunehmen, wobei die Maschenweite der innersten Lage so eng gehalten ist, daß einwachsendes Knochengewebe und Kunststoff voneinander getrennt bleiben.

Ein - beispielsweise aus Titan oder einer Titanlegierung bestehendes - mehrlagiges Metallnetz, das vorzugsweise ein Drahtgewebe ist und bei dem die Drähte in ihren Querschnitten jederzeit und/oder die Maschenweiten so gewählt werden können, dass sie das Implantat nicht unzulässig versteifen, ermöglicht über seine ganze Fläche eine gleichmässige Porosität sowohl knochen- wie kunststoffseitig; diese Porosität ergibt durch das Aufeinanderschichten mehrerer Lagen darüberhinaus eine definierte und kontrollierbare Abnahme der knochenseitigen Porengrössen von außen nach innen, die schliesslich so eng werden, dass die Lage mit den engsten Poren sowohl für das einwachsende Knochengewebe als auch für den eindringenden Kunststoff "undurchdringlich" wird.

Die Einbettung einer Lage des Drahtnetzes in die Kontaktfläche des Kunststoffes, der vorzugsweise eine der in der Implantat-Technik bekannten und vielfach verwendeten Polyäthylen-Modifikationen ist, erfolgt in einfacher Weise dadurch, dass diese Lage in den durch erhöhte Temperatur erweichten Kunststoff durch erhöhten Druck eingepresst wird. Die Temperaturerhöhung ist dabei auf Temperaturen unterhalb der Kristallitschmelztemperatur (135 - 140° C) begrenzt; der erforderliche Druck ist abhängig von der Struktur des Netzes und von der gewählten Temperatur. Er kann beispielsweise bis zu etwa 390 bar betragen.

Aus medizinischen Gründen ist die Maschenweite der innersten Lage so eng gehalten, dass einwachsendes Knochengewebe und Kunststoff voneinander getrennt bleiben. Es hat sich weiterhin bei Drahtgeweben als zweckmässig erwiesen, wenn die Drähte mindestens der eingebetteten Lagen zu einer verbreiterten Auflagefläche abgeflacht sind, wobei diese Abflachung beispielsweise durch Walzen vor oder nach dem Aufeinanderschichten der einzelnen Lage erreicht werden kann. Für die gegenseitige Haftung werden die Lagen - einschliesslich der später vom Kunststoff umschlossenen Lage - nach dem Aufeinanderschichten mit Vorteil einem Sinterprozess (Diffusionsschweissung) unterworfen, durch den mindestens in örtlichen Teilbereichen eine metallurgische Bindung entsteht.

Im folgenden wird die Erfindung anhand eines Ausführungsbeispiels im Zusammenhang mit der Zeichnung näher erläutert.

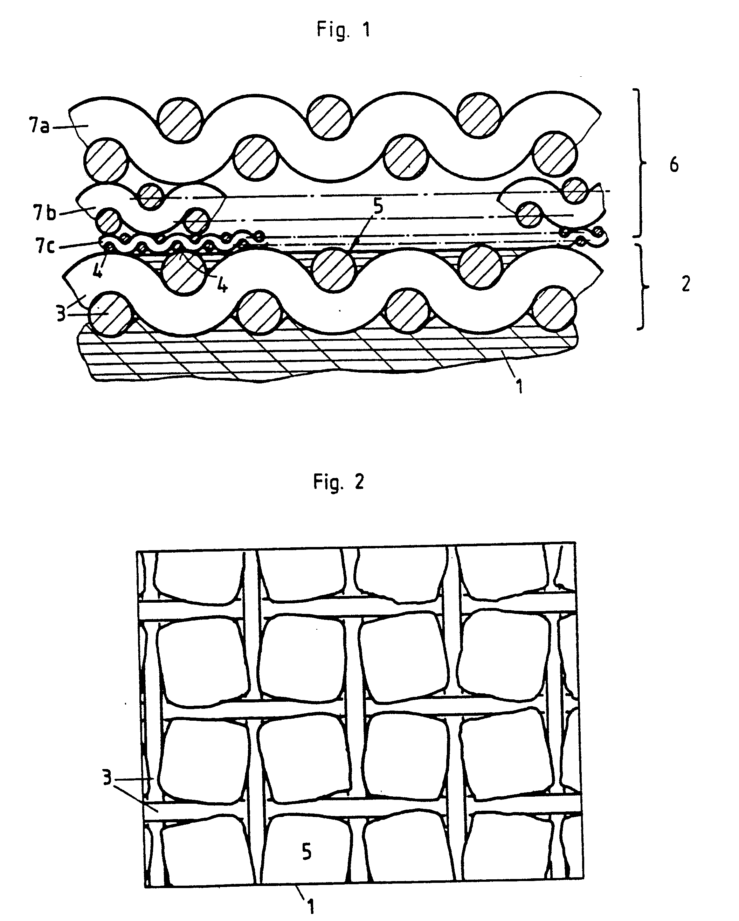

Fig. 1    ist ein Querschnitt durch ein Ausführungsbeispiel einer nur in einem Ausschnitt vergrössert dargestellten Implantatoberfläche;

Fig. 2    stellt ebenfalls in einem vergrösserten Ausschnitt eine Aufsicht auf ein von Kunststoff umschlossenes Metallnetz dar.

In das Implantat 1, das beispielsweise eine Hüftgelenkspfanne aus Polyäthylen ist, ist eine Lage eines Metallnetzes 2 eingebettet, das aus einem Gewebe aus einzelnen Metalldrähten 3 besteht. Durch diese eingebettete Lage 2, deren Scheitelbereiche 4 in dem gezeigten Beispiel leicht aus der Ober- oder Kontaktfläche 5 des Implantates 1 vorstehen, ist das Implantat 1 so fest mit dem Metallnetz verbunden, dass Relativbewegungen zwischen beiden, und damit Abrieb, praktisch vermieden werden.

Auf der Lage 2, deren Maschenweiten und Draht-"Durchmesser" beispielsweise zwischen 0,2 und 1,5 mm betragen, ist eine Auflage 6 angeordnet. Diese besteht von innen nach aussen aus drei Lagen oder Schichten 7, die ebenfalls Drahtgewebe sind. Durchmesser und Maschenweite nehmen von innen nach aussen zu, so dass sich die Porengrösse der einzelnen Lage der Auflage 6 stetig, d.h. ohne sich in einer Zwischenlage zu verringern, und definiert vergrössert. So hat die innerste Lage 7c beispielweise Drähte mit einem Durchmesser von 0,1 mm und eine Maschenweite des Gewebes von 0,2 mm; in der mittleren Lage 7b sind die entsprechenden Werte 0,25 mm und 0,5 mm, während sie bei der äussersten Lage 7a 0,5 mm und 1 mm betragen.

Die Auflage 6 besteht aus einem der als besonders gewebefreundlich bekannten Metalle wie Titan, Tantal, Niob, Zirkon oder Legierungen mit diesen Metallen als Basiswerkstoffen. Sie bilden somit eine gewebefreundliche Oberflächenstruktur, in die im Laufe der Zeit Knochengewebe einwächst, wodurch das Implantat fest und knochenzementfrei im Knochen verankert wird. Die Maschenweite der innersten Lage 7c der Auflage ist dabei so eng, dass durch sie kein Gewebe hindurchwächst; diese Lage bewirkt daher eine vollständige Abschirmung und Trennung zwischen Knochen und Kunststoff.

## Patentansprüche

1. Knochenimplantat aus Kunststoff, insbesondere Hüftgelenkspfanne, dessen den Knochen kontaktierende Oberfläche (5), mindestens teilweise mit einer eine zementfreie Verankerung fördernden, porösen Struktur versehen ist, dadurch gekennzeichnet, dass die poröse Struktur eine mehrlagige Maschenware (2, 6) aus Metalldraht mit in jeder Lage (2, 7) definierten und regelmässigen Maschenweiten ist, wobei mindestens eine Lage (2) in die Kontaktfläche (5) des Kunststoffimplantates (1) mindestens teilweise eingebettet ist und auf der mindestens teilweise eingebetteten Lage (2) mehrere Lagen (7) der gleichen Maschenware (2, 6) angeordnet sind, deren Maschenweiten von Lage zu Lage (7c, 7b, 7a) nach aussen zunehmen, wobei die Maschenweite der innersten Lage (7c) so eng gehalten ist, dass einwachsendes Knochengewebe und Kunststoff (1) voneinander getrennt bleiben.

2. Knochenimplantat nach Anspruch 1, dadurch gekennzeichnet, dass die Drähte (3) mindestens der eingebetteten Lage (2) zu einer verbreiterten Auflagefläche abgeflacht sind.

## Claims

1. A plastics bone implant, more particularly an acetabulum, whose bone-contacting surface (5) has at least to some extent a porous structure promoting uncemented fixing, characterised in that the porous structure is a multilayer mesh material (2, 6) of metal wire, the material having defined and regular mesh sizes in each layer (2, 7), at least one layer (2) being embedded at least to some extent in the contact surface (5) of the plastics implant and a number of layers (7) of the same mesh material (2, 6) being disposed on the at least partly embedded layer (2), the mesh sizes of the layers (7) increasing outwardly from layer to layer (7c, 7b, 7a), the mesh size of the innermost layer (7c) being so small that the Invading bone tissue and the plastics (1) remain separated from one another.

2. A bone implant according to claim 1, characterised in that the wires (3) of at least the embedded layer (2) are flattened to form a widened on lay surface.

## Revendications

1. Implant osseux en matière plastique, en particulier cuvette cotyloïde d'articulation de la hanche, dont la surface (5) en contat avec l'os est pourvue, au moins partiellement, d'une structure poreuse favorisant un ancrage sans ciment, caractérisé en ce que la structure poreuse est une toile maillée multicouche (2, 6) en fil métallique, dont chaque couche (2, 7) présente des largeurs de maille définies et régulières, au moins une couche (2) de la surface (5) de contact de l'implant (1) en matière plastique étant au moins partiellement encastrée et plusieurs couches (7) de la même toile maillée (2, 6) étant disposées sur la couche (2) au moins partiellement encastrée, couches (7) dont les largeurs de mailles, d'une couche à l'autre (7c, 7b, 7a) augmentent vers l'extérieur, la largeur de maille de la couche (7c) située le plus à l'intérieur étant maintenue si serrée que le tissu osseux, croissant en profondeur, et la matière plastique (1) restent séparés l'un de l'autre.

2. Implant osseux selon la revendication 1, caractérisé en ce que les fils (3), au moins dans la couche encastrée (2), sont aplatis de façon à présenter une surface d'appui élargie.

Fig. 1

Fig. 2